# EUROPÄISCHE PATENTANMELDUNG

(11) **EP 1 835 287 A2**
(43) Veröffentlichungstag der Anmeldung: **19.09.2007**
(21) Anmeldenummer: 07104001.8
(22) Anmeldetag: 13.03.2007
(51) Int. Cl.: G01N 33/68, G01N 33/74, G01N 33/94

(54) **Verfahren und Testbestecke zur Bestimmung von neuroendokrinen gesundheitlichen Störungen**

(30) Priorität: 14.03.2006 DE 102006012171; 04.08.2006 DE 102006038224
(71) Anmelder: Bieger, Wilfried P., 80336 München (DE)
(72) Erfinder: Bieger, Wilfried P., 80336 München (DE)
(74) Vertreter: Wehlan, Helmut

(57) **Zusammenfassung**

Die Erfindung betrifft Verfahren und Testbestecks zur Bestimmung von neuroendokrinen gesundheitlichen Störungen, die mit Stresshormon- und /oder Neurotransmitter-Ungleichgewichten des neuroendokrinen Regelkreises einhergehen. Typische Erkrankungen dieser Art sind etwa die zentrale Fatigue (Erschöpfung), das Burn-Out- oder Sickness-Syndrom, die multiple Chemikalienüberempfindlichkeit (MCS), Migräne, das Prämenstruelle Syndrom (PMS), Schlafstörungen oder Appetitstörungen. Die erfindungsgemäße Lösung macht den Nachweis von Neurosteroiden oder Stresshormonen möglich und dient der Analyse und Erforschung von Neurostresszuständen durch Bestimmung von Glucocorticoiden, Sexualsteroiden und Aminosäuren /Neurotransmittern bzw. ihren Metaboliten in Körperflüssigkeiten (Speichel, Serum, Urin).

## Beschreibung

Die Erfindung betrifft Verfahren und Testbestecks zur Bestimmung von neuroendokrinen gesundheitlichen Störungen, die mit Stresshormon- und /oder Neurotransmitter-Ungleichgewichten des neuroendokrinen Regelkreises einhergehen.

Zu diesen Störungen / Erkrankungen gehören etwa die zentrale Fatigue (Erschöpfung), das Chronische Müdigkeitssyndrom (Chronic Fatigue Syndrom - CFS), die multiple Chemikalienüberempfindlichkeit (multiple chemical sensitivity - MCS), CMI (Chronic Multisystem Illnesses; Jason LA et al. Chronic fatigue syndrome, fibromyalgia, and multiple chemical sensitivities in a community-based sample of persons with chronic fatigue syndrome-like symptoms. Psychosom Med 2000; 62:655-63), primäre und sekundäre Depressionen, Burn-Out-Syndrom oder Sickness-Syndrom (Larson et al. 2001, Brain Behaviour Immunity, 15:371-387), Fibromyalgie, Migräne, Klaustrophobie, Schlafstörungen, Prämenstruelles Syndrom (PMS), Essstörungen / Appetitstörungen, Heißhunger, Kohlehydrat-Heißhunger (Craving), Übergewicht / Adipositas, Sick Building Syndrom (SBS), Building related illness, Enviromental somatisation syndrome, Idiopathic Environmental Intolerance, Kopfschmerzen, Muskelschmerzen, Konzentrationsstörungen, Gedächtnisschwäche (Kurzzeitgedächtnis), Koordinationsstörungen oder Reizkolon.

Patienten mit dem Beschwerdebild einer multiplen Chemikalienüberempfindlichkeit (multiple chemical sensitivity - MCS) etwa zeigen bei geringer Chemikalienexposition ein obligates systemisches Beschwerdebild. Die betroffenen Personen klagen über Gedächtnisstörungen, Schwäche, Schwindel, Kopfschmerzen, Schweißausbrüche, Atemnot, Zittern, Gelenk- und Muskelschmerzen o.ä.. Die Reaktion wird durch verschiedenste Substanzen (z.B. Autoabgase, Parfüme, Farben, Lacke, Holzschutzmittel) ausgelöst, meist unabhängig von deren chemischer Struktur und bei sehr niedrigen Konzentrationen, die normalerweise auf den Menschen keine subjektiv wahrnehmbaren Auswirkungen haben.

Diese ungewöhnlichen Reaktionen wurden im Laufe der letzten 20 Jahre mit Begriffen wie Sick Building Syndrom (SBS), Enviromental somatisation syndrome, Building related illness o.ä. umschrieben. Weder sind bis heute die Ursachen dieses Krankheitsbildes bekannt, noch gibt es gesicherte diagnostische Parameter dafür. In der Regel lassen sich bei den betroffenen Personen keine erhöhten Chemikalienspiegel nachweisen. Es sind weder neurologische oder internistische Auffälligkeiten feststellbar, die klassischen Laborparameter sind ebenfalls normal. Ebenso wenig gibt es Anhaltspunkte dafür, dass allergische Reaktionen eine Rolle spielen. Immer wieder diskutiert wurde u.a. eine Hyperreaktivität des Immunsystems als Ursache dieses Krankheitsbildes, wobei bisher keine experimentellen oder klinischen Anhaltspunkte für diese Hypothese aufgezeigt werden konnten.

Nach Ashford, N.A. (Toxicology and Industrial Health, 1999, Nr. 3 S. 1-7 und dort zitierte Dokumente) umfasst die Sensitivität gegenüber Chemikalien drei recht unterschiedliche Kategorien:
1) Die Reaktion normaler Versuchspersonen auf bekannte Expositionen entsprechend der herkömmlichen Dosis-Wirkung-Beziehung. Zu dieser Kategorie gehören Reaktionen von Personen am unteren Ende der Verteilung klassischer Reaktionen auf toxische Stoffe innerhalb der Grundgesamtheit sowie die klassische Allergie und andere Formen einer immunologisch vermittelten Sensitivität.
2) Die Reaktion normaler Versuchspersonen auf bekannte oder unbekannte Expositionen, die sich mit klassischen oder bekannten Mechanismen nicht erklären lassen. Zu dieser Kategorie gehören:
   a) das Sick Building Syndrome (SBS), bei dem Menschen auf bekannte oder unbekannte Expositionen reagieren, deren Symptome aber verschwinden, wenn sie der vom Gebäude ausgehenden Belastung nicht ausgesetzt sind; und
   b) die Sensitivität, wie sie beispielsweise durch Toluoldiisocyanat induziert wird, die zunächst als spezifische Überempfindlichkeit für einen einzelnen Stoff (oder eine Stoffgruppe) auftritt, sich aber zu einer unspezifischen Überempfindlichkeit entwickeln kann, wie sie unter der nachstehenden Kategorie 3) beschrieben ist.
3) Die erhöhte übermäßige oder ungewöhnliche Reaktion von Personen auf bekannte oder unbekannte Expositionen, bei denen die Symptome nicht vollständig verschwinden, wenn die Belastung nicht mehr gegeben ist, und/oder bei denen sich die "Sensitivität" auf andere Stoffe auszudehnen scheint. Diese Personen können folgendes zeigen:
   a) eine erhöhte Reaktion auf Stoffe bei gleicher Expositionsdosis im Vergleich zu anderen Personen;
   b) eine Reaktion bei niedrigeren Dosen als diejenigen, die andere Personen beeinträchtigen; und/oder
   c) eine Reaktion zu einem früheren Zeitpunkt als dem für andere Personen zutreffenden Zeitpunkt.

Cullen ("Der Internist", Heft 1/98, S. 105 ff.) definiert MCS wie folgt:
- die Symptome wurden im Zusammenhang mit einer dokumentierbaren Umweltexposition erworben
- die Symptome betreffen mehr als ein Organ
- die Symptome erscheinen und verschwinden in Zusammenhang mit vorhersehbaren Stimuli
- die Symptome werden durch Chemikalien unterschiedlicher Struktur und Wirkmechanismus hervorgerufen
- die Expositionen müssen sehr niedrig sein, im Bereich von 1 % der üblichen Schwellendosis
- kein einzelner Organfunktionstest kann die Symptome erklären.

Das Sick-Building-Syndrom wird auf der Internetseite
(http://www.medizinfo.com/umweltmedizin/syndrome/sickbuilding.htm) folgendermaßen charakterisiert: Symptome und Verlauf sind Kopfschmerzen, Müdigkeit, Konzentrationsschwäche, Depression, Vergesslichkeit, Empfindungsstörungen an Händen, Füßen, Armen und Beinen, unklare Schmerzen, Reizerscheinungen an Augen, Atemwegen und Haut. Die Symptome klingen im Freien ab und treten in geschlossenen Räumen erneut auf. Ursachen können überheizte Räume, niedrige Luftwechselraten in geschlossenen Räumen, Schimmelpilzsporen, chemische Ausdünstungen, oft auch Klimaanlagen, welche die Luft nur recyceln und nicht erneuern, sein.

Bisher wurde keine somatische Ursache bzw. ein entsprechendes diagnostisches Kriterium für MCS gefunden, so dass vielfach dazu geneigt wird, MCS als psychosomatische Gesundheitsstörung einzustufen.

In den letzten Jahren konnten diagnostisch erstmals Unterschiede zwischen MCS-Patienten, Normalpersonen und Personen mit Depressionen oder anderen psychischen Auffälligkeiten festgestellt werden.

Bisherige diagnostische Feststellungen der MCS oder ähnlicher Erkrankungen umfassen z.B. folgendes:
- Bei Patienten mit Chemikaliensensibilität / -intoleranz wurde demonstriert, dass sie über eine erhöhte nasale Reaktivität verfügen gegenüber Normalpersonen, selbst in der Ruhephase (Doty, Deems, Frye, Pelberg, Shapiro - Olfactory sensitivity, nasal resistence and autonomic function in patients with multiple chemical sensitivities. Arch Otolaryngol Head Neck Surg. 1988)
- Neurogene Inflammation in Verbindung mit dem Peptid Substanz P./ Multisystem-Inflammation bei Chemikaliensensitiven (Meggs - Neurogenic inflammation and sensitivity to environmental chemicals. Environ. Health Perspect. 1995)
- Kognitive Abnormalitäten bei der visuellen Erinnerungsfähigkeit bei den Personen mit MCS, die gravierende Veränderungen in ihrer Lebensführung durchführen mussten (Fiedler, Kipen, DeLuca, Kelly-McNeil, Natelson - A controlled comparison of multiple chemical sensitivity and chronic fatigue syndrome, Psychosom. Med. 1996)
- Höhere corticale EEG-Aktivierung (geringere Alphawellen und größere Betawellen Aktivität) bei hochgradig Chemikaliensensiblen (Schwartz, Bell, Dikman, et al. - EEG responses to low level chemicals in normals and cacosmics, Toxicol Ind Health. 1994)
- Perfusionsstörungen durch SPECT-Scans festgestellt, Antikörper gegen Chemikalien (Heuser, Wodani - Multiple chemical Sensitivities: Addendum to biologic Markers in Immunotoxicology National Research Council, National Accademy Press 1992)
- Störungen des Glucosestoffwechsels im Gehirn durch PET festgestellt (Kuklinski - Glutathiontransferasen und Krankheit, ZFU 1999)
- Abnormale Immunzellpopulation, besonders Anstieg von TH1 nach Chemikalienexposition, Autoimmunantikörper (Rea - Chemical Sensitivity Vol. I-IV, 1995)
- Porphyrie, Porphyrinopathie (Donnay, Ziem - Protocol for evaluating disorders of porphyrin metabolism in chemically sensitive patients, MCS Referral & Resources, 1995)
- Abnormale Plasma β-Endorphinwerte (Bell, Bootzin, Davis, Hau, Rithenbaugh, Johnson, Schwartz - Time-Depentent Sensitization of plasma beta-endorphin in community elderly with self-reported environmental chemical odor intolerance, Society of Biological Psychiatry 1996)
- Abnormales Serum Neopterin (Bell, Pataraca, Baldwin, Klimas, Schwartz, Hardin - Serum Neopterin and Somatization in women with chemical intolerance, depressives and normals, Neuropsychobiology 1998).

Das Chronic Fatigue Syndrome (CFS) ist ein klinisch definiertes Krankheitsbild, das durch schwere, lähmende Erschöpfung mit mehr als 50% Einschränkung der normalen Leistungsfähigkeit und eine Kombination von Symptomen charakterisiert wird, bei denen Konzentrationsschwäche und Merkfähigkeitsstörung, Schlafstörungen, Muskel- und Gliederschmerzen im Vordergrund stehen (Holmes GP, Kaplan JE, Gantz NM, Komaroff AL, Schonberger LB, Straus SE et al., Chronic fatigue syndrome: a working case definition, Ann Intern Med. 1988; 108:387-9; Prins JB, van der Meer JWM, Bleijenberg G, Chronic Fatigue Syndrome, Lancet 2006; 367:346-355).

Spezifische - medizinisch-wissenschaftlich gesicherte - diagnostische Marker zum Nachweis eines CFS gab es bisher nicht.

Da auch bei anderen Krankheiten bestimmte Symptome - wie ausgeprägte Müdigkeit, Kopfschmerzen u.a. - auftreten können, ist eine sorgfältige Ausschlussdiagnostik bezogen auf bekannte Krankheiten (Differentialdiagnostik) erforderlich (Informationen der Bundesweiten Selbsthilfegruppe für Patienten mit MCS / CFS-Syndrom e.V., Bayreuth /Germany). Durch Laboruntersuchungen müssen deshalb z.B. Entzündungen, Tumore usw. ausgeschlossen werden. Funktionsprüfungen aller inneren Organe, des Pankreas, der Niere, der Leber, des Darms, der Schilddrüse und der Nebenniere sind durchzuführen. Schwere Infektionen, reaktivierte / chronische Virusinfektionen, Autoimmunerkrankungen, Tumorerkrankungen, Immuntherapie mit Zytokinen bei Tumoren oder Virusinfekten, Erkrankungen endokriner Organe wie Schilddrüse, Nebenniere, Hypophyse, Diabetes mellitus können ein ähnliches Müdigkeitssyndrom verursachen.

In der Patentliteratur ist die Diagnose von Krankheiten wie CFS mehrfach beschrieben worden. So wird gemäß der Offenlegungsschrift WO 93/13419 die Anwesenheit des Zytokins Interleukin-1 als Diagnosemarker für CFS verwendet. Auch der lösliche Interleukin-2-Rezeptor, ein Indikator für die Anwesenheit von Interleukin-2, wird als Diagnosemarker eingesetzt (WO93/14226). In der Schrift WO97/14963 wird ein Testkit zur Erkennung von CFS bzw. Fibromyalgie auf der Basis eines antipolymeren Antikörpers beschrieben. Eine Diagnose von CFS durch Analyse der peripheren Blutzellen ist Gegenstand des US-Patents 5,538,856. Eine positive Diagnose von CFS liegt dann vor, wenn in CD8+-Zellen die Zell-Marker CD38 oder HLA-DR zunehmen oder die CD11b-Marken abnehmen. Das Dokument WO 96/30759 berichtet über eine Methode zur Diagnose von CFS mittels statistischer Untersuchungen an roten Blutzellen.

Weiterhin wird in der Patentliteratur über die Bestimmung eines etwa 30 kDa großen RNase L-Moleküls berichtet (US 5,985,565), das aus peripheren Blutzellen extrahiert wird. Im US-Patent 5,853,996 wird beschrieben, dass in peripheren Blut-Monozyten zur Diagnose von CFS die p68-Kinase-Aktivität, mRNA-Levels, Apoptose und Zell-Zyklus-Analysen bestimmt werden und mit den Werten von gesunden Individuen verglichen werden. Die Diagnose von CFS mittels des 2'-5'A/ RNase P-pathway, einschließlich der Messung des 2'-5'A Oligonucleotid-Levels in peripheren Leukozyten ist Gegenstand der Schrift WO 91/00097.

Auch die Bestimmung eines neuen CFIDS (Chronic Fatigue Immunodysfunction Syndrome) Virus wurde zur Diagnose von CFIDS vorgeschlagen (WO 92/05760). Andere Dokumente berichten über die Bestimmung der spezifischen Dopamin-beta-Hydroxylase-Aktivität (WO 98/58076) oder über Zusammenhänge zwischen Variationen im Arginin-Vasopressin-Receptor-Gen-2 (AVPR2; WO 98/37239).

Die Zytokinsynthese der inflammatorischen und antiinflammatorischen Immunantwort unterliegt einer neuroendokrinen Feed-Back-Regulation. So ist die neuroendokrine Stressreaktion (Cortisol) unmittelbar mit entzündlichen Mechanismen (Anstieg von IFN-gamma, IL1β, IL6, TNF-alpha) assoziiert. Zytokinbestimmungen sind routinegängige Labormethoden, publiziert u.a. von Asadullah et al. 1997, Dtsch.med.Wschr 122:1424-1431.

Zu den wichtigsten Mediatoren der pathologischen Stress-Antwort wird das inflammatorische Zytokin IL1 gerechnet (Konsman et al, Trends Neurosci 25: 154-159, 2002). Es besteht demnach ein neuroendokrin-immunologischer Regelkreis: Nervenzellen weisen Rezeptoren für Zytokine wie IL-1 oder TNF-alpha auf, und umgekehrt verfügen Immunzellen über Rezeptoren für biogene Amine wie Adrenalin, Noradrenalin, Dopamin oder Serotonin (Haas, Schauenstein, Allergy 56:470-477, 2001). So stimuliert IL1 die Freisetzung von Cortisol, IFN-gamma hemmt die Serotoninsynthese über die Aktivierung des Enzyms IDO (Indolamin-2,3-Dioxygenase) mit verstärktem Abbau von Tryptophan zu Kynurenin (Munn et al, J Exp Med, 189: 1363-1372, 1999). Unter chronischem Stress nimmt die Ausschüttung von biogenen Aminen ab, auch durch die eingeschränkte Verfügbarkeit von Vorläufermolekülen wie Tyrosin (4-Hydroxy-phenylalanin) oder Tryptophan (Indolyl-(3)-alanin). Erkrankungen mit pathologischer Stress-Response wie das Burn-Out-Syndrom oder die sog. Sickness-Behaviour gehen deshalb mit einer inflammatorischen Dysregulation der Immunabwehr einher (Larson et al. 2001, Brain Behaviour Immunity,15:371-387).

In zahlreichen Publikationen sind wiederholt Zusammenhänge zwischen Fatigue, CFS, FMS, MCS (Abkürzungsverzeichnis hinter den Beispielen) und anderen umweltassoziierten Gesundheitsstörungen sowie pathologischen Veränderungen der Neurostresshormone CRH (Corticotropin-Relasing Hormon), Cortisol, DHEAS, Prolactin, Wachstumshormon, Melatonin und der Neurotransmitter Katecholamine, Serotonin, PEA beschrieben worden (Friedman EM, Lawrence DA, Environmental stress mediates changes in neuroimmunological interactions; Toxicol Sci 2002; 67:4-10).

Der Zusammenhang zwischen Stress, Depression, Motivation, Kognition, Aufmerksamkeit, Schlaf, Ängsten, Klaustrophobie (krankhafte Furcht vor geschlossenen Räumen), Appetitverhalten, Energiestoffwechsel, Schmerzempfindungen einerseits und neuroendokrinen Regelmechanismen andererseits ist Gegenstand intensiver Forschung. Hypercortisolismus gilt als das zentrale biochemische Kriterium der primären Depression, Burn-Out als pathologische Spätmanifestation des chronischen Stress (Gold PW, Chrousos GP; Organization of the stress system and its dysregulation in melancholic and atypical depression: high versus low CRH/NE states, Mol Psychiatry 2002; 7: 254-75; de Kloet ER, Joels M, Holsboer F; Stress and the brain: from adaptation to disease. Nat Rev 2005; 6:463-75; Charmandari E, Tsigos C, Chrousos G: Endocrinology of the stress response, Annu Rev Physiol 2005;67:259-84).

Zentrale Fatigue gilt als Hauptkriterium des CFS und zahlreicher durch Aktivierung proinflammatorischer Mechanismen geprägter Erkrankungen wie chronische Infektionen, Autoimmunerkrankungen, Tumoren, umwelttoxische Syndrome oder Folge von Therapie mit proinflammatorischen Zytokinen und Mediatoren (Chaudhuri A, Behan PO, Fatigue in neurological disorders, Lancet 2004; 363: 978-88; Patarca R, Cytokines and chronic fatigue syndrome Ann NY Acad Sci 2002: 185-202).

Der Zusammenhang zwischen Stress, Stress-verwandten Krankheiten und Inflammation wird als zentrales Element der genannten Krankheitsprozesse gesehen (Turnbull AT, Rivier CL, Regulation of the hypothalamic-pituitary-adrenal axis by cytokines: actions and mechanisms of action. Physiol Rev 1999; 79:1-83).

Die Neurosteroidhormone und die Neurotransmitter agieren in enger Wechselbeziehung. Aktivierung der Neurohormonachse führt zur Aktivierung der Neurotransmitter, insbesondere der biogenen Amine - und umgekehrt. Synthese, Abbau und Aktion auf Rezeptorebene sind bei den Neurotransmittern eng miteinander vernetzt (O'Connor TM, O'Halloran DJ, Shanahan F, The stress response and the hypothalamic-pituitary-adrenal axis: from molecule to melancholia, QJ Med 2000, 93:323-33; van Praag HM, Crossroads of corticotropin releasing hormone, corticosteroids and monoamines, Neurotox Res 2002; 4:531-55).

Der Nachteil der beschriebenen Lösungen liegt darin, dass es sich jeweils um eine Ausschlussdiagnostik handelt, die zwar andere Krankheiten ausschließen kann, aber keinen gesicherten Hinweis auf MCS, CFS, SBS o.a. gesundheitliche Störungen liefert. Auch wurde bisher angenommen, dass beispielsweise aufgrund der Unterschiede zwischen MCS und SBS eine einheitliche Diagnose überhaupt nicht möglich ist.

Der Erfindung liegt die Aufgabe zugrunde, die Nachteile der bekannten Lösungen zu beseitigen und neue Möglichkeiten zur Diagnose von neuroendokrinen gesundheitlichen Störungen, insbesondere von Neurotransmitter-Ungleichgewichten des neuroendokrinen Regelkreises, bereitzustellen.

Die Aufgabe wurde dadurch gelöst, dass Verfahren entwickelt wurden, denen der Nachweis von Neurosteroiden und /oder Stresshormonen sowie von Neurotransmittern bzw. ihren Metaboliten in Körperflüssigkeiten zugrunde liegt. Die erfindungsgemäßen Verfahren zur Bestimmung von neuroendokrinen gesundheitlichen Störungen bestehen darin, dass Glucocorticoide, Sexualsteroide und Aminosäuren /Neurotransmitter und /oder ihre Metaboliten in Körperflüssigkeiten gemessen werden.

Die erfindungsgemäßen Testbestecks dienen der Analyse und Erforschung von Neurostresszuständen durch Bestimmung von Glucocorticoiden, Sexualsteroiden und Neurotransmittern bzw. ihren Metaboliten in Körperflüssigkeiten. Sie bestehen aus Immunoassays und /oder Anordnungen für die Flüssigkeitschromatographie /HPLC. Diese Testkits stellen Analysekits für medizinische und veterinärmedizinische Zwecke dar. Sie bestehen aus biochemischen Reagenzien für die Analyse von Neurostresszuständen aufgrund von Neuroregulationsstörungen mit Stresshormon- und /oder Neurotransmitter-Defiziten, -Überschüssen bzw. -Störungen des neuroendokrinen Regulationsgleichgewichts. Sie bestehen in ELISA(Enzyme Linked Immunosorbent Assays)-Tests oder in chromatographischen Nachweisverfahren (HPLC - High Pressure Liquid Chromatographie, LC-MSMS - Liquid Chromatographie mit Massenspektrometerdetektion in zwei Schritten) zum Nachweis von Neurosteroiden und Stresshormonen im Speichel und in anderen Körperflüssigkeiten sowie von Neurotransmittern bzw. ihren Metaboliten im Urin und in anderen Körperflüssigkeiten (Speichel, Serum).

Überraschenderweise hat sich herausgestellt, dass durch den Nachweis von Neurosteroiden und /oder Stresshormonen im Speichel sowie von Neurotransmittern bzw. ihren Metaboliten im Urin und in anderen Körperflüssigkeiten (Speichel, Serum) Rückschlüsse auf neuroendokrine Störungen des neurovegetativen Regulationsgleichgewichts und die damit verbundenen Erkrankungen möglich sind.

Die Messung der Steroidhormone im Speichel ist seit Jahren Standard. Sie beruht auf dem Nachweis der Steroide mit spezifischen, markierten Antikörpern im ELISA- oder RIA-Verfahren (ELISA: Enzyme Linked Immunosorbent Assay, Enzym-Immunoassay; RIA: Radioimmunoassay).

Speichelcortisol ist hochgradig korreliert mit dem freien Cortisol und dem Gesamtcortisol im Serum, in seiner Aussagekraft sogar der Serumbestimmung überlegen (Aardal-Eriksson E, Karlberg BE, Holm AC, Salivary cortisol - an alternative to serum cortisol determinations in dynamic function tests; Clin Chem Lab Med 1998; 36:215-22; Putigano P, Dubini A, Toja P et al.: Salivary cortisol measurement in normal-weight, obese and anorexic women: comparison with plasma cortisol; Eur J Endocrinol 2001; 145:165-71). Das gleiche gilt für die anderen Steroidhormone wie DHEA (Dehydroepiandrosteron), DHEA(S) bzw. DHEAS (Dehydroepiandrosteron(sulfat)), Testosteron, Östradiol, Progesteron, Östron, Androstendion, deren freier, nichteiweißgebundener Anteil auf Grund des lipophilen Charakters die Speicheldrüsenendothelien frei passieren kann und mit den Serumwerten hochgradig korreliert ist [(Tschöp M, Behre HM, Nieschlag E et al.: A time-resolved fluorescence immunoassay for the measurement of testosterone in saliva: monitoring of testosterone replacement therapy with testosterone buciclate, Clin Chem Lab Med. 1998; 36:223-30); Hucklebridge F et al.: The diurnal patterns of the adrenal steroids cortisol and dehydroepiandrosterone (DHEA) in relation to awakening, Psychoneuroendocrinology, 2005, 30:51-7; Shirtcliff EA et al.: Use of salivary biomarkers in biobehavioral research: cotton-based sample collection methods can interfere with salivary immunoassay results Psychoneuroendocrinology, 2001 Feb, 26(2): 165-73; Lipson S, Ellison P: Development of protocols for the application of salivary steroid analysis to field conditions, Am J Hum Biol 1989; 1:249-55)]. Die Messung beruht auf dem Nachweis der Steroide mit spezifischen, markierten Antikörpern im ELISA oder RIA-Verfahren.

Im Unterschied zu den Steroidhormonen im Speichel ist die Messung der Neurotransmitter im Urin als Marker der neuroendokrinen Regulation nur partiell untersucht. Die Messung beruht auf dem Nachweis der Neurotransmitter /Metaboliten mit spezifischen, markierten Antikörpern im ELISA- oder RIA-Verfahren oder alternativ auf der HPLC (Hochdruckflüssigkeitschromatographie, High Performance / High Pressure Liquid Chromatographie).

Seit langem etabliert ist die Bestimmung von Serotonin (5-Hydroxy-tryptamin) und den Katecholaminen als Tumormarker endokriner Tumoren /Malignome. Die Messung beruht auf dem Nachweis der Neurotransmitter /Metaboliten mit spezifischen, markierten Antikörpern im ELISA- oder RIA-Verfahren oder alternativ auf der HPLC.

Histamin wird im Zusammenhang mit Allergien im Urin untersucht. Glutamin, Glutamat, Taurin, Glycin und GABA werden im Rahmen der Aminosäureanalyse in Serum und Urin bestimmt. Für PEA (Phenylethylamin) liegen einzelne Berichte zur Urinmessung bei Depression oder ADS /ADHS vor (Kusaga A et al.: Increased urinary phenylethylamine after methyphenidate treatment in children with ADHD, Ann Neurol 2002; 52:372-4).

Die Messung beruht auf dem Nachweis der Aminosäuren /Neurotransmitter mit spezifischen, markierten Antikörpern im ELISA- oder RIA-Verfahren oder alternativ auf der HPLC.

Die Plasmabestimmung der biogenen Amine ist wegen ihrer sehr kurzen Plasmahalbwertzeit von wenigen Minuten nur für Extremzustände geeignet. Die Messung beruht auf dem Nachweis der Aminosäuren /Neurotransmitter mit spezifischen, markierten Antikörpern im ELISA- oder RIA-Verfahren oder alternativ auf der HPLC.

Die Bewertung der Urinkonzentrationen in Zusammenhang mit der Neuroregulation ist bisher nur vereinzelt erfolgt [Hughes JW et al., Depression and anxiety symptoms are related to increased 24 hour urinary norepinephrine excretion among healthy middle-aged women, J Psychosom Res 2004, 57:353-8; Lykouras L et al., Association of biogenic amine metabolites with symptomatology in delusional (psychotic) and nondelusional depressed patients, Biol Psychiatry 1995; 19:877-87; Meguid MM, Fetissov SO, Varma M, Sato T, Zhang L, Laviano A, Rossi-Fanelli F.: Hypothalamic dopamine and serotonin in the regulation of food intake, Nutrition 2000, 16:843-57; Human ageing and the sympathoadrenal system Seals DR, Esler MD. J Physiol 2000;528:407-17; The sympathetic nerve-an integrative interface between two supersystems: the brain and the immune system. Elenkov IJ, Wilder RL, Chrousos GP, Vizi ES. Pharmacol Rev 2000 Dec;52(4):595-638; Seasonal and biological variation of urinary epinephrine, norepinephrine, and cortisol in healthy women, Hansen AM, Garde AH, Skovgaard LT, Christensen JM. Clin Chim Acta 2001 Jul 5;309(1):25-35)].

Erfindungsgemäß werden Speichelmessungen von Cortisol (im Tagesprofil), von DHEA (Dehydroepiandrosteron), Serotonin (5-Hydroxy-tryptamin), Melatonin (5-Methoxy-N-acetyltryptamin), gegebenenfalls auch von Testosteron, 17-OH-Progesteron, Substanz P und / oder von Katecholaminen vorgenommen.

Die vorzunehmenden Urinbestimmungen beziehen sich auf Katecholamine (Adrenalin, Noradrenalin, Dopamin), gegebenenfalls auch auf ihre Metaboliten Metanephrin, Normetanephrin, Vanillinmandelsäure (VMA), 3-Methoxy-4-Hydroxy-Phenylglykol (MHPG), Dopaminmetabolit (DOPAC: 3,4-Dihydroxyphenylacetic acid), des weiteren auf die Aminosäuren / Neurotransmitter Glutamat, Glutamin, Gamma-Aminobuttersäure (GABA), Glycin, Histamin, Taurin, Phenylethylamin (PEA) und auf Serotonin. Die Messung beruht auf dem Nachweis der Aminosäuren /Neurotransmitter mit spezifischen, markierten Antikörpern im ELISA- oder RIA-Verfahren oder alternativ auf der HPLC.

Die erfindungsgemäßen Testbestecks eignen sich zur Bestimmung von neuroendokrinen gesundheitlichen Störungen und damit von Neurostresszuständen, von Neuroregulationsstörungen, von Störungen der Hypothalamus-Hypophysen-Nebennierenrindenachse und /oder neuroinflammatorischen Zuständen der genannten Erkrankungen.

Diese Testbestecks bestehen aus mindestens 3 Speichelröhrchen und 1 bis 2 Urinröhrchen (und gegebenenfalls Serum-Röhrchen) mit Stabilisator (EDTA, HCl, Na-Metabisulfit), Sammelschale und Pipette, Sammelanleitung, Etiketten, standardisiertem Anamnese- und Untersuchungsbogen sowie den in den ELISA- oder RIA-Verfahren oder der HPLC benötigten spezifischen, markierten Antikörpern.

Die Messung der Neurotransmitter wird einerseits mittels Enzymimmunoassay vorgenommen, so die Katecholamine, PEA, Serotonin, Histamin bzw. Methylhistamin, Melatonin, GABA oder Glutamat; andererseits mittels HPLC nach Derivatisierung, so für Glycin, Glutamin, Glutaminsäure und /oder Taurin.

Mit den erfindungsgemäßen Verfahren und Testbestecks können gesundheitliche Störungen, Neurostresszustände, Neuroregulationsstörungen bzw. Regulationsstörungen der neuroendokrinen Stressachse wie
a) Chronisches Müdigkeitssyndrom (CFS bzw. CFIDS)
b) Multiple Chemikalienüberempfindlichkeit (multiple chemical sensitivity MCS)
c) CMI (Chronic Multisystem Illnesses)
d) Depressionen
e) Burn-Out-Syndrom
f) Fibromyalgie
g) Migräne
h) Schlafstörungen
i) Prämenstruelles Syndrom (PMS)
j) Essstörungen / Appetitstörungen, Heißhunger, Kohlehydrat-Heißhunger
k) Übergewicht / Adipositas
l) Craving
m) Sick-Building Syndrome (SBS)
n) Building related illness
o) Environmental somatisation syndrome
p) Idiopatic Environmental Intolerance
q) Kopfschmerzen
r) Gelenk-, Muskelschmerzen
s) Konzentrationsstörungen, Gedächtnisschwäche (Kurzzeitgedächtnis)
t) Aufmerksamkeitsdefizitstörungen (ADS, ADHS)
u) Koordinationsstörungen
v) Reizkolon
w) Nahrungsmittelunverträglichkeiten
x) Klaustrophobie
y) Regulationsstörungen der neuroendokrinen Stressachse, wie z.B. das Burn-Out-Syndrom oder das Sickness-Syndrom
überraschend klar getestet werden.

Die erfindungsgemäßen Verfahren zur Bestimmung von neuroendokrinen gesundheitlichen Störungen bestehen darin, dass das Cortisoltagesprofil und DHEA bzw. DHEAS, Testosteron, Östradiol, gegebenenfalls auch Progesteron, 17-Hydroxyprogesteron und Neurotransmitter (Katecholamine, Serotonin, etc.) in Körperflüssigkeiten wie Serum, Speichel und / oder Urin gemessen werden.

Die erfindungsgemäße Diagnostik von neuroendokrinen Erkrankungen erfolgt durch Testung von Cortisol oder Corticosteron in Körperflüssigkeiten und DHEA /DHEAS im Speichel, Cortisoltagesprofil im Speichel, Melatonin im Speichel und /oder im Morgenurin, Serotonin im Urin, Serum und /oder im Speichel, und Neurotransmitter wie Serotonin, Adrenalin (3,4-Dihydroxy-phenyl-1-ethanol-methylamin, int. Bez.: Epinephrin), Noradrenalin (3,4-Dihydroxyphenyl-1-ethanol-amin), Dopamin (3,4-Dihydroxy-β-phenethylamin), Glutamat, GABA, PEA, Glycin, Taurin, Glutamin, Histamin, Aspartat und gegebenenfalls Neurotransmittermetabolite wie DOPAC, MHPG, Metanephrine, VMS (Vanillinmandelsäure) oder 5-HIES (5-Hydroxyindolessigsäure, ein Serotoninmetabolit) im Urin (2. Morgenurin).

Das erfindungsgemäße Verfahren zur Diagnostik /Bestimmung von CFS (Chronisches Müdigkeitssyndrom, Chronic Fatigue Syndrom) besteht darin, dass Cortisol im Tagesprofil sowie DHEA(S), Melatonin, Serotonin, Katecholamine, GABA und /oder Glutamat gemessen werden.

Die erfindungsgemäße Diagnostik /Bestimmung von MCS erfolgt dadurch, dass Cortisol im Tagesprofil sowie DHEA(S), Melatonin, Serotonin, Substanz P, Katecholamine, GABA, Glutamat, Histamin und /oder Glycin gemessen werden.

Eine weitere Ausführungsform der erfinderischen Verfahren besteht darin, das zur Bestimmung von Fibromyalgie Cortisol im Tagesprofil sowie DHEA(S), Melatonin, Serotonin, Katecholamine, GABA und /oder Glutamat gemessen werden.

Weitere Ausführungsformen der Erfindung bestehen darin, dass zur Diagnostik
- der zentralen Fatigue (Erschöpfung) Cortisol im Tagesprofil sowie DHEA(S), Melatonin, Serotonin, Katecholamine, GABA, Glutamat und /oder Histamin
- von Aufmerksamkeitsdefizitstörungen (ADS, ADHS) Serotonin, Katecholamine, GABA, Glutamat, PEA, Histamin und /oder Kryptopyrrol
- von Depressionen Cortisol im Tagesprofil sowie DHEA(S), Melatonin, Serotonin, Katecholamine, GABA, Glutamat, PEA, Testosteron und /oder Östradiol und gegebenenfalls Progesteron
- des Burn-Out-Syndroms Cortisol im Tagesprofil sowie DHEA(S), Melatonin, Serotonin, Katecholamine, GABA, Glutamat und /oder Testosteron
- von Schlafstörungen Cortisol im Tagesprofil sowie DHEA(S), Melatonin, Serotonin, Katecholamine, GABA, Glutamat, Histamin, Testosteron, und /oder Östradiol
- von Migräne Cortisol im Tagesprofil sowie DHEA(S), Melatonin, Serotonin, Katecholamine, PEA, GABA, Glutamat, Histamin und /oder Glycin
- von Essstörungen Cortisol im Tagesprofil sowie DHEA(S), Serotonin, Katecholamine, PEA, Histamin und /oder Glycin
- der Adipositas Cortisol im Tagesprofil sowie DHEA(S), Testosteron, Östradiol, Melatonin, Serotonin, Katecholamine, GABA, Glutamat und /oder Histamin
- von PMS, dem Prämenstruellen Syndrom, Cortisol im Tagesprofil und DHEA(S), Testosteron, Östradiol, Progesteron, Melatonin, Serotonin und /oder Katecholamine
- gemessen werden.

Die Messung beruht auf einer kombinierten, unblutigen Messung von Steroidhormonen im Speichel und von Neurotransmittern im Urin unter Verwendung spezieller Testbestecks mit Stabilisierung der Messparameter zur Feststellung neuroendokriner Funktionsstörungen.

Den erfindungsgemäßen Testbestecks zur Bestimmung /Diagnostik von neuroendokrinen gesundheitlichen Störungen liegen Immunoassays zugrunde, mit deren Hilfe das Cortisoltagesprofil und DHEA bzw. DHEAS (Abkürzungsverzeichnis hinter den Beispielen), Testosteron, Östradiol, gegebenenfalls auch Progesteron, 17-Hydroxyprogesteron und Neurotransmitter (Katecholamine, Serotonin, etc.) in Körperflüssigkeiten wie Serum, Speichel und / oder Urin gemessen werden.

Die Diagnostik von neuroendokrinen Erkrankungen mit Hilfe der erfindungsgemäßen Testbestecks erfolgt durch Bestimmumg von Cortisol oder Corticosteron in Körperflüssigkeiten und DHEA /DHEAS im Speichel, Cortisoltagesprofil im Speichel, Melatonin im Speichel und /oder im Morgenurin, Serotonin im Urin, Serum und /oder im Speichel, und Neurotransmitter wie Serotonin, Adrenalin (3,4-Dihydroxy-phenyl-1-ethanol-methylamin, int. Bez.: Epinephrin), Noradrenalin (3,4-Dihydroxy-phenyl-1-ethanol-amin), Dopamin (3,4-Dihydroxy- -phenethylamin), PEA, GABA, Glutamat, Glycin, Taurin, Glutamin, Histamin, Methylhistamin, Aspartat und gegebenenfalls Neurotransmittermetabolite wie DOPAC, MHPG, Metanephrine, VMS (Vanillinmandelsäure) oder 5-HIES (5-Hydroxyindolessigsäure, ein Serotoninmetabolit) im Urin (2. Morgenurin).

Die erfindungsgemäßen Testbestecks bestehen aus Immunoassays und /oder Anordnungen für die Flüssigkeitschromatographie /HPLC zur Messung von Glucocorticoiden, Sexualsteroiden und Neurotransmittern und /oder ihren Metaboliten in Körperflüssigkeiten.

Die Bestimmung von Cortisol oder von Corticosteron erfolgt mittels Chemolumineszenz- oder Enzymimmunoassay, die Bestimmung von DHEA /DHEAS, Testosteron, Östradiol oder 17-Hydroxyprogesteron mittels Chemolumineszenz-, Enzym- oder Radioimmunoassay, von Serotonin, den Katecholaminen Dopamin, Adrenalin und Noradrenalin sowie von GABA, Glutamat, PEA, Histamin bzw. Methyhistamin, Glycin, Taurin, der Metaboliten MHPG, DOPAC, VMS, 5-HIES oder Metanephrine mittels Enzym- bzw. Radioimmunassay oder Flüssigkeitschromatographie.

Die erfindungsgemäßen Testbestecks bestehen aus Immunoassays und /oder Anordnungen für die Flüssigkeitschromatographie /HPLC zur Messung /Bestimmung der Glucocorticoide Cortisol oder Corticosteron im Speichel, der Sexualsteroide DHEA, DHEAS, Testosteron, Östradiol, Progesteron oder 17-Hydroxyprogesteron und der Neurotransmitter Katecholamine und ihre Metaboliten, Aminosäuren und ihre Metaboliten im Serum, Speichel oder Urin.

In näherer Bestimmumg bestehen die Testbestecks aus Immunoassays und /oder Anordnungen für die Flüssigkeitschromatographie /HPLC zur Messung /Bestimmung der Katecholamine Adrenalin, Noradrenalin oder Dopamin und ihrer Metaboliten Metanephrin, Normetanephrin, Vanillinmandelsäure oder 3-Methoxy-4-Hydroxy-Phenylglykol und zur Bestimmung /Messung der Aminosäuren und ihrer Metaboliten Serotonin, Melatonin, Glutamat, Glutamin, GABA, Glycin, Taurin oder Phenylethylamin.

Die erfindungsgemäßen Testbestecks eignen sich des weiteren zur Bestimmung /Messung der Glucocorticoide, der Sexualsteroide und von Substanz P im Speichel, von Melatonin im Speichel und /oder im Morgenurin, Serotonin im Urin, Serum und /oder im Speichel, und zur Bestimmung /Messung der Neurotransmitter Serotonin, Adrenalin, Noradrenalin, Dopamin, Glutamat, GABA, PEA, Glycin, Taurin, Glutamin, Histamin, Methylhistamin, Aspartat und der Neurotransmittermetabolite DOPAC, MHPG, Metanephrine, VMS oder 5-HIES im Urin oder im zweiten Morgenurin.

Die verwendeten Assays stellen Chemolumineszenz-, Enzym- oder Radioimmunoassays dar. Sie bestehen aus Immunoassays zur Bestimmung /Messung der Steroide im Speichel und aus Immunoassays oder aus Anordnungen für die Flüssigkeitschromatographie /HPLC zur Bestimmung /Messung von Aminosäuren /Neurotransmittern und ihren Metaboliten im Urin, Serum oder im Speichel.

Die Testbestecks zur Messung der Neurotransmitter bestehen einerseits aus Enzymimmunoassays zur Bestimmung /Messung der Katecholamine, PEA, Serotonin, Histamin, Methylhistamin, Melatonin, GABA oder Glutamat, andererseits aus Anordnungen für die Flüssigkeitschromatographie /HPLC zur Bestimmung /Messung von Glycin und /oder Taurin nach Derivatisierung. Die Messung von Melatonin erfolgt im Speichel und /oder im Morgenurin und von Serotonin im Serum, Urin und /oder Speichel.

Speichelmessungen werden vorgenommen von Cortisol, von DHEA(s), Serotonin, Melatonin, Substanz P, Testosteron, 17-OH-Progesteron und /oder von Katecholaminen.

In speziellen Ausführungsformen betreffen die Testbestecks die Diagnostik des Chronischen Müdigkeitssyndroms (CFS, Chronic Fatigue Syndrom). Sie bestehen in diesem Fall aus Assays zur Bestimmung /Messung der Steroide im Speichel und aus Immunoassays oder aus Anordnungen für die Flüssigkeitschromatographie /HPLC zur Bestimmung /Messung von Aminosäuren /Neurotransmittern und ihren Metaboliten im Urin, Serum oder Speichel. Im einzelnen bestehen sie aus Chemolumineszenz- oder Enzymimmunoassays zur Bestimmung /Messung von Cortisol im Tagesprofil, aus Chemolumineszenz-, Enzym- oder Radioimmunoassays zur Bestimmung von DHEA /DHEAS und von Testosteron, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA und /oder Glutamat. Bei der Bewertung der Messungen ergibt sich, dass die Cortisolwerte deutlich erniedrigt sind, die 17-OH-Progesteron normal bis niedrig, Serotonin stark erniedrigt, Melatonin und GABA oft niedrig, Noradrenalin erhöht bis stark erniedrigt, Dopamin, Adrenalin und Glutamat normal bis erniedrigt und der Testosteron-Wert niedrig sein kann.

In einer weiteren Ausführungsform betreffen die Testbestecks die Diagnostik der multiplen Chemikalienüberempfindlichkeit (multiple chemical sensitivity, MCS). In diesem Fall bestehen sie aus Assays zur Messung der Steroide im Speichel und aus Immunoassays oder aus Anordnungen für die Flüssigkeitschromatographie /HPLC zur Bestimmung /Messung von Aminosäuren /Neurotransmittern und ihren Metaboliten im Urin, Serum oder Speichel, darunter von Glycin nach Derivatisierung im Urin oder im zweiten Morgenurin mittels Enzym- /Radioimmunassay oder HPLC /Flüssigkeitschromatographie. Im einzelnen bestehen sie aus Chemolumineszenz- oder Enzymimmunoassays zur Bestimmung von Cortisol oder Corticosteron im Tagesprofil, aus Chemolumineszenz-, Enzym- oder Radioimmunoassays zur Bestimmung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Bestimmung von Serotonin, Substanz P, Katecholaminen, GABA, Glutamat, Histamin und /oder Glycin in Körperflüssigkeiten, darunter das Cortisol-/ Corticosterontagesprofil und DHEA(S) im Speichel, Melatonin im Speichel und /oder im Morgenurin, Serotonin im Urin, Serum und /oder im Speichel.

Zur Diagnostik der Fibromyalgie werden Testbestecks vorgeschlagen, die aus Chemolumineszenz- oder Enzymimmunoassays zur Messung von Cortisol im Tagesprofil, aus Chemolumineszenz-, Enzym- oder Radioimmunoassays zur Bestimmung /Messung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA und /oder Glutamat bestehen.

In einer weiteren Ausführungsform betreffen die Testbestecks die Diagnostik der Fatigue (zentrale Erschöpfung). In diesem Fall bestehen sie aus Chemolumineszenz- oder Enzymimmunoassays zur Messung von Cortisol im Tagesprofil, aus Chemolumineszenz-, Enzym- oder Radioimmunoassays zur Bestimmung /Messung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA, Glutamat und /oder Histamin.

Die erfindungsgemäßen Testbestecks eignen sich auch zur Bestimmung von Aufmerksamkeitsdefizitstörungen (ADS, ADHS). In diesem Fall bestehen sie aus Chemolumineszenz- oder Enzymimmunoassays zur Messung von Cortisol im Tagesprofil, aus Enzym- oder Radioimmunassays oder aus Anordnungen für die Flüssigkeitschromatographie /HPLC zur Bestimmung /Messung von Serotonin, Katecholaminen, GABA, Glutamat, PEA, Histamin und einer photometrischen Anordnung zur Bestimmung /Messung von Kryptopyrrol.

In einer weiteren Ausführungsform betreffen die Testbestecks die Diagnostik von Depressionen. In diesem Fall bestehen sie aus Chemolumineszenz- oder Enzymimmunoassays zur Messung von Cortisol im Tagesprofil, aus Chemolumineszenz-, Enzym- oder Radioimmunoassays zur Bestimmung /Messung von DHEA /DHEAS und Testosteron, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA, Glutamat und /oder PEA.

Die erfindungsgemäßen Testbestecks eignen sich des weiteren zur Diagnostik des Bum-Out-Syndroms. Für diesen Fall bestehen sie aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS und Testosteron, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA und /oder Glutamat.

In einer weiteren Ausführungsform betreffen die Testbestecks die Diagnostik von Schlafstörungen. In diesem Fall bestehen sie aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS, Testosteron und /oder Östradiol, aus Enzymimmunoassays zur Bestimmung von Melatonin, aus Enzym- oder Radioimmunassays oder aus Anordnungen für die Flüssigkeitschromatographie /HPLC zur Bestimmung von Serotonin und Katecholaminen.

Weitere Testbestecks betreffen die Diagnostik von Migräne, wobei sie in diesem Fall aus Chemolumineszenz- oder Enzymimmunoassays zur Bestimmung von Cortisol im Tagesprofil bestehen, des weiteren aus Chemolumineszenz-, Enzym- oder Radioimmunoassays zur Bestimmung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, PEA, GABA, Glutamat, Histamin und /oder Glycin.

Zur Diagnostik von Essstörungen werden mit dieser Erfindung Testbestecks vorgeschlagen, die aus Chemolumineszenz- oder Enzymimmunoassays zur Bestimmung von Cortisol im Tagesprofil, aus Chemolumineszenz-, Enzym- oder Radioimmunoassays zur Bestimmung /Messung von DHEA /DHEAS, aus Enzym- oder Radioimmunassays oder aus Anordnungen für die Flüssigkeitschromatographie /HPLC zur Bestimmung /Messung von Serotonin, Katecholaminen, PEA, Histamin und /oder Glycin bestehen.

In einer weiteren Ausführungsform betreffen die Testbestecks die Diagnostik der Adipositas. In diesem Fall bestehen sie aus Chemolumineszenz- oder Enzymimmunoassays zur Bestimmung von Cortisol im Tagesprofil, aus Chemolumineszenz-, Enzym- oder Radioimmunoassays zur Bestimmung /Messung von DHEA /DHEAS, Testosteron und /oder Östradiol, aus Enzymimmunoassays zur Bestimmung /Messung von Melatonin, aus Enzym- oder Radioimmunassays oder aus Anordnungen für die Flüssigkeitschromatographie /HPLC zur Bestimmung /Messung von Serotonin, Katecholaminen, GABA, Glutamat und /oder Histamin.

Weitere Testbestecks betreffen die Diagnostik des Prämenstruellen Syndroms (PMS). Sie bestehen aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, Testosteron, Östradiol Progesteron, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin und /oder Katecholaminen.

In weiteren Ausführungsformen betreffen die Testbestecks die Bestimmung neuroendokriner gesundheitlicher Störungen. In diesen Fällen bestehen sie aus Immunoassays zur Messung der Steroide-Werte im Speichel und aus Immunoassays oder aus Anordnungen für die Flüssigkeitschromatographie /HPLC zur Bestimmung von Aminosäuren /Neurotransmittern und ihren Metaboliten im Urin, Serum oder Speichel zur Erkennung von Krankheiten / Störungen der Art
a) Chronisches Müdigkeitssyndrom (CFS bzw. CFIDS)
b) Multiple Chemikalienüberempfindlichkeit (multiple chemical sensitivity MCS)
c) CMI (Chronic Multisystem Illnesses)
d) Depressionen
e) Burn-Out-Syndrom
f) Fibromyalgie
g) Migräne
h) Schlafstörungen
i) Prämenstruelles Syndrom (PMS)
j) Essstörungen / Appetitstörungen, Heißhunger, Kohlehydrat-Heißhunger
k) Übergewicht / Adipositas
l) Craving
m) Sick-Building Syndrome (SBS)
n) Building related illness
o) Environmental somatisation syndrome
p) Idiopatic Environmental Intolerance
q) Kopfschmerzen
r) Gelenk-, Muskelschmerzen
s) Konzentrationsstörungen, Gedächtnisschwäche (Kurzzeitgedächtnis)
t) Aufmerksamkeitsdefizitstörungen (ADS, ADHS)
u) Koordinationsstörungen
v) Reizkolon
w) Nahrungsmittelunverträglichkeiten
x) Klaustrophobie
y) Regulationsstörungen der neuroendokrinen Stressachse, wie z.B. das Burn-Out-Syndrom oder das Sickness-Syndrom.

Damit eignen sich die erfindungsgemäßen Testbestecks zur Bestimmung von neuroendokrinen gesundheitlichen Störungen sowie von Neurotransmitter-Störungen und damit verbundener Neuroregulationsstörungen.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht in einer Kombination aus bekannten (Bestimmung von Katecholaminen, Aminosäuren oder Neurotransmittern) und neuen Elementen (etwa Cortisoltagesprofil im Speichel; Bestimmung von Neurotransmittern im 2. Morgenurin; Testbestecks zur Bestimmung von Neurostresszuständen), die sich gegenseitig beeinflussen und in ihrer neuen Gesamtwirkung einen Gebrauchsvorteil und den erstrebten Erfolg ergeben, der darin liegt, dass erstmals eine positive Diagnose von neuroendokrinen gesundheitlichen Störungen - im Unterschied zur Ausschlussdiagnostik - bereitgestellt wird. Damit ist ein Nachweisverfahren gefunden worden, mit dem Neurotransmitter-Störungen und damit verbundene Neuroregulationsstörungen biochemisch verifiziert und dann spezifisch behandelt werden können.

Die Erfindung soll anhand von Ausführungsbeispielen erläutert werden, ohne auf diese Beispiele beschränkt zu sein.

### Ausführungsbeispiele

### Beispiel 0: Bestimmung von neuroendokrinen gesundheitlichen Störungen

Von Patienten mit derartigen Störungen werden Glucocorticoide, Sexualsteroide und Aminosäuren /Neurotransmitter sowie ihre Metaboliten in Körperflüssigkeiten erfasst.

Als Glucocorticoide werden Cortisol oder Corticosteron im Speichel, als Sexualsteroide DHEA, DHEAS, Testosteron, Östradiol, Progesteron oder 17-Hydroxyprogesteron und als Neurotransmitter Katecholamine und ihre Metaboliten, Aminosäuren und ihre Metaboliten im Serum, Speichel oder Urin gemessen.

Als Katecholamine werden Adrenalin, Noradrenalin oder Dopamin und als ihre Metaboliten Metanephrin, Normetanephrin, Vanillinmandelsäure oder 3-Methoxy-4-Hydroxy-Phenylglykol und als Aminosäuren und ihre Metaboliten Serotonin, Melatonin, Glutamat, Glutamin, GABA, Glycin, Taurin oder Phenylethylamin erfasst.

Die Testung der Glucocorticoide, der Sexualsteroide und von Substanz P erfolgt im Speichel, von Melatonin im Speichel und /oder im Morgenurin, von Serotonin im Urin, Serum und /oder im Speichel, und der Neurotransmitter Serotonin, Adrenalin, Noradrenalin, Dopamin, Glutamat, GABA, PEA, Glycin, Taurin, Glutamin, Histamin, Methylhistamin, Aspartat sowie der Neurotransmittermetabolite DOPAC, MHPG, Metanephrine, VMS oder 5-HIES im Urin oder im zweiten Morgenurin.

Als Neurotransmitter werden Melatonin im Speichel und /oder im Morgenurin und Serotonin im Serum, Urin und /oder Speichel gemessen.

Von Cortisol, DHEA (Dehydroepiandrosteron), Serotonin, Melatonin, Substanz P, gegebenenfalls auch von Testosteron, 17-OH-Progesteron und /oder von Katecholaminen werden Speichelmessungen vorgenommen.

Die Messung der Neurotransmitter erfolgt mittels Enzymimmunoassays, so die der Katecholamine, PEA, Serotonin, Histamin, Methylhistamin, Melatonin, GABA oder Glutamat und für Glycin, Glutamin, Glutaminsäure und /oder Taurin mittels HPLC nach Derivatisierung.

### Bewertung:

Die ermittelten Werte an Glucocorticoiden, Sexualsteroiden und Aminosäuren /Neurotransmittern ermöglichen Rückschlüsse auf das Vorliegen oder auf ein Nichtvorliegen einer Erkrankung / neuroendokrinen Störung.

Dabei wird von folgenden Werten ausgegangen:

### NORMWERTE:

| | | |
|---|---|---|
| Cortisol 8.00 Uhr: | | 4-12 pg/ml |
| | 12.00 Uhr: | 1,5 . 5,0 pg/ml |
| | 20.00 Uhr: | 0,4-1,5 pg/ml |
| DHEAS (Speichel) | | 0,4 - 5,0 ng/ml. |

Die Urinwerte werden auf die Kreatininkonzentration (Krea) im Urin bezogen (und so standardisiert):

| | |
|---|---|
| Serotonin | 175 - 225 mcg/g Krea |
| Adrenalin | 8-12 mcg/g Kreatinin |
| Noradrenalin | 30-55 mcg/g Krea |
| Dopamin | 175-250 mcg/g Krea |
| Histamin | 10-25 mcg/g Krea |
| GABA | 1,5 - 5,0 µmol/g Krea |
| Glutamat | 8-25 µmol/g Krea |
| Glycin | 200 - 400 µmol/g Krea |
| Melatonin (Speichel) | 20 - 60 pg/ml |
| Melatonin (Morgenurin) | 58 - 85 pg/ml. |

### Testbestecks:

Sie bestehen aus Immunoassays und /oder Anordnungen für die Flüssigkeitschromatographie /HPLC zur Messung von Glucocorticoiden, Sexualsteroiden und von Aminosäuren /Neurotransmittern und /oder ihren Metaboliten in Körperflüssigkeiten. Dabei erfolgt die Messung der Steroide im Speichel und der Aminosäuren / Neurotransmittern im Urin, Serum oder Speichel.

Sie bestehen aus Speichel-, Urin- und /oder Serum-Röhrchen, die Stabilisatoren - EDTA, HCl, Na-Metabisulfit - enthalten, ferner aus Sammelschalen, Pipetten, Sammelanleitungen, Etiketten, standardisiertem Anamnese- und Untersuchungsbogen und aus den in den ELISA- oder RIA-Verfahren, der Chemolumineszenz oder der HPLC benötigten spezifischen, markierten Antikörpern.

### Beispiel 1: MCS (multiple chemical sensitivity)

Das Verfahren zur Diagnostik der multiplen Chemikalienüberempfindlichkeit (multiple chemical sensitivity, MCS), besteht darin, dass
- Cortisol oder Corticosteron und ferner DHEA /DHEA(S), Melatonin, Serotonin, Substanz P, Katecholamine, GABA, Glutamat, Histamin und /oder Glycin in Körperflüssigkeiten
- Cortisol im Tagesprofil und DHEA /DHEA(S), Melatonin, Serotonin, Katecholamine, GABA und /oder Glutamat
- das Cortisoltagesprofil und DHEA /DHEA(S) im Speichel, Melatonin im Speichel und /oder im Morgenurin, Serotonin im Urin, Serum und /oder im Speichel
- gemessen werden.

### Bewertung:

Bei MCS-Patienten findet man gegenüber Normalpersonen unter Belastung (Umweltstoffe, volatile Stoffe) einen Cortisolabfall. Substanz P im Speichel fällt ab. Serotonin ist häufig erniedrigt, Noradrenalin erhöht, Dopamin normal. Histamin ist in einem Teil der Fälle erhöht.

### Testbestecks:

Sie bestehen aus Chemolumineszenz- oder Enzymimmunoassays zur Messung /Bestimmung von Cortisol oder Corticosteron im Tagesprofil, aus Chemolumineszenz-, Enzym- oder Radioimmunoassays zur Bestimmung von DHEA /DHEAS, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Substanz P, Katecholaminen, GABA, Glutamat, Histamin und /oder Glycin in Körperflüssigkeiten, darunter das Cortisol-/ Corticosterontagesprofil und DHEA(S) im Speichel, Melatonin im Speichel und /oder im Morgenurin, Serotonin im Urin, Serum und /oder im Speichel.

### Beispiel 2: CFS (Chronic Fatigue Syndrom)

Das Verfahren zur Diagnostik von CFS (Chronisches Müdigkeitssyndrom) besteht darin, dass Cortisol im Tagesprofil und DHEA /DHEA(S), Melatonin, Serotonin, Katecholamine, GA-BA und /oder Glutamat gemessen werden.

### Bewertung:

Bei dem CFS ist Cortisol im Tagesprofil deutlich erniedrigt, 17-OH-Progesteron normal bis niedrig, Serotonin stark erniedrigt, Melatonin und GABA oft niedrig, Noradrenalin erhöht bis stark erniedrigt, Dopamin, Adrenalin und Glutamat normal bis erniedrigt, Testosteron kann niedrig sein.

### Testbestecks:

Die Testbestecks zur Diagnostik von CFS bestehen aus Chemolumineszenz- oder Enzymimmunoassays zur Bestimmung von Cortisol im Tagesprofil, das deutlich erniedrigte Werte ausweist und zur Bestimmung von Testosteron, dessen Werte niedrig sein können, aus Chemolumineszenz-, Enzym- oder Radioimmunoassays zur Bestimmung von DHEA /DHEAS mit normalen bis niedrigen Werten für 17-OH-Progesteron, aus Enzymimmunoassays zur Bestimmung von Melatonin, das oft niedrige Werte ausweist, aus Enzym- oder Radioimmunassays oder aus Anordnungen für die Flüssigkeitschromatographie /HPLC zur Bestimmung von Serotonin - mit oft stark erniedrigten Werten, Katecholaminen - mit erhöhten bis stark erniedrigten Werten für Noradrenalin und normalen bis erniedrigten Werten für Dopamin, Adrenalin, GABA und Glutamat.

### Beispiel 3: Depressionen

Das Verfahren zur Diagnostik von Depressionen besteht darin, dass Cortisol im Tagesprofil und DHEA /DHEA(S), Melatonin, Serotonin, Katecholamine, GABA, Glutamat, PEA, Testosteron und /oder Östradiol gemessen werden.

### Bewertung:

Cortisol ist hoch, DHEA(S) normal bis erhöht, Serotonin niedrig, Noradrenalin, PEA erhöht.
Der Hypercortisolismus ist vielfach als anerkanntestes biochemisches Kriterium der primären Depression beschrieben worden, auch der Serotoninmangel und gegebenenfalls PEA- und Noradrenalinmangel.

### Testbestecks:

Die Testbestecks zur Diagnostik von Depressionen bestehen aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS und Testosteron, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA, Glutamat und /oder PEA.

### Beispiel 4: Burn-out-Syndrom

Das Verfahren zur Diagnostik des Burn-Out-Syndroms besteht darin, dass Cortisol im Tagesprofil und DHEA /DHEA(S), Melatonin, Serotonin, Katecholamine, GABA, Glutamat und /oder Testosteron gemessen werden.

### Bewertung:

Cortisol ist sehr niedrig, die Tagesrhythmik aufgehoben, DHEA(S) ist normal bis niedrig, Serotonin sehr niedrig, Noradrenalin und Glutamat normal bis erhöht, Melatonin niedrig, Testosteron normal bis erniedrigt.

### Testbestecks:

Die Testbestecks zur Diagnostik des Burn-Out-Syndroms bestehen aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS und Testosteron, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA und /oder Glutamat.

### Beispiel 5: Fibromyalgie

Das Verfahren zur Diagnostik der Fibromyalgie besteht darin, dass Cortisol im Tagesprofil und DHEA /DHEA(S), Melatonin, Serotonin, Katecholamine, GABA und /oder Glutamat gemessen werden.

### Bewertung:

Cortisol ist niedrig normal, DHEA(S) unauffällig, Serotonin erniedrigt, die Katecholamine niedrig normal, Melatonin niedrig, Glutamat normal bis erhöht, GABA normal bis erhöht.

### Testbestecks:

Zur Diagnostik der Fibromyalgie finden Testbestecks Verwendung, die aus Chemolumineszenz- oder Enzymimmunoassays zur Messung /Bestimmung von Cortisol im Tagesprofil, aus Chemolumineszenz-, Enzym- oder Radioimmunoassays zur Bestimmung von DHEA /DHEAS, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA und /oder Glutamat bestehen.

### Beispiel 6: Fatigue (zentrale Erschöpfung)

Das Verfahren zur Diagnostik der Fatigue (zentrale Erschöpfung) besteht darin, dass Cortisol im Tagesprofil und DHEA /DHEA(S), Melatonin, Serotonin, Katecholamine, GABA, Glutamat und /oder Histamin gemessen werden.

### Bewertung:

Cortisol ist erniedrigt, DHEA(S) meist normal, Serotonin deutlich erniedrigt, Dopamin normal bis erniedrigt, Noradrenalin normal, erhöht oder niedrig, Adrenalin normal bis niedrig, GABA tendenziell erhöht, Glutamat normal, Histamin normal bis erhöht.

### Testbestecks:

Die Testbestecks zur Diagnostik der Fatigue (zentrale Erschöpfung) bestehen aus Chemolumineszenz- oder Enzymimmunoassays zur Messung /Bestimmung von Cortisol im Tagesprofil, aus Chemolumineszenz-, Enzym- oder Radioimmunoassays zur Bestimmung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Messung /Bestimmung von Serotonin, Katecholaminen, GABA, Glutamat und /oder Histamin.

### Beispiel 7: Schlafstörungen

Das Verfahren zur Diagnostik von Schlafstörungen besteht darin, dass Cortisol im Tagesprofil und DHEA /DHEA(S), Melatonin, Serotonin, Katecholamine, Histamin, Testosteron und /oder Östradiol gemessen werden.

### Bewertung:

Cortisol ist normal, niedrig oder hoch; DHEA(S) ist niedrig bis normal, Serotonin ist niedrig, Melatonin ist niedrig oder im Peak abgeschwächt, Noradrenalin erhöht, Adrenalin niedrig bis normal, Histamin erhöht bis normal, Testosteron und/oder Östradiol normal bis niedrig.

### Testbestecks:

Die Testbestecks zur Diagnostik von Schlafstörungen bestehen aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS, Testosteron und /oder Östradiol, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin und Katecholaminen.

### Beispiel 8: Aufmerksamkeitsdefizitstörungen (ADS, ADHS)

Die Verfahren zur Diagnostik von Aufmerksamkeitsdefizitstörungen (ADS, ADHS) bestehen darin, dass Serotonin, Katecholamine, GABA, Glutamat, PEA, Histamin und /oder Kryptopyrrol gemessen werden.

### Bewertung:

(Cortisol ist normal), Kryptopyrrol ist erhöht, Serotonin niedrig, Noradrenalin hoch oder erniedrigt, Adrenalin normal, Dopamin normal, GABA normal bis erhöht, PEA niedrig, Histamin normal bis hoch.

### Testbestecks:

Die Testbestecks zur Diagnostik von Aufmerksamkeitsdefizitstörungen (ADS, ADHS), bestehen aus Chemolumineszenz- oder Enzymimmunoassays zur Bestimmung von Cortisol im Tagesprofil, aus Enzym- oder Radioimmunassays oder aus Anordnungen für die Flüssigkeitschromatographie /HPLC zur Messung /Bestimmung von Serotonin, Katecholaminen, GABA, Glutamat, PEA, Histamin und einer photometrischen Anordnung zur Bestimmung /Messung von Kryptopyrrol.

### Beispiel 9: Migräne

Das Verfahren zur Diagnostik von Migräne besteht darin, dass Cortisol im Tagesprofil und DHEA /DHEA(S), Melatonin, Serotonin, Katecholamine, PEA, GABA, Glutamat, Histamin und /oder Glycin gemessen werden.

### Bewertung:

Cortisol ist normal, DHEA(S) ebenfalls normal, Serotonin niedrig, Melatonin oft erniedrigt, Noradrenalin hoch, Adrenalin normal, Dopamin normal, PEA hoch, GABA normal, Glutamat normal bis erhöht, Histamin normal bis hoch, Glycin normal bis erniedrigt.

### Testbestecks:

Die Testbestecks zur Diagnostik von Migräne bestehen aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin, Katecholaminen, PEA, GABA, Glutamat, Histamin und /oder Glycin.

### Beispiel 10: Essstörungen

Das Verfahren zur Diagnostik von Essstörungen besteht darin, dass Cortisol im Tagesprofil und DHEA /DHEA(S), Serotonin, Katecholamine, PEA, Histamin, Glycin und /oder GABA gemessen werden.

### Bewertung:

Cortisol ist normal bis erhöht, DHEA(S) normal, Serotonin niedrig, Noradrenalin normal bis erhöht, Dopamin niedrig bis normal, PEA hoch, Histamin normal bis erhöht, Glycin niedrig bis normal, GABA normal bis niedrig.

### Testbestecks:

Die Testbestecks zur Diagnostik von Essstörungen bestehen aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, aus Enzym- oder Radioimmunassays oder aus Anordnungen für die Flüssigkeitschromatographie /HPLC zur Messung /Bestimmung von Serotonin, Katecholaminen, PEA, Histamin und /oder Glycin.

### Beispiel 11: Adipositas

Das Verfahren zur Diagnostik der Adipositas besteht darin, dass Cortisol im Tagesprofil und DHEA /DHEA(S), Testosteron, Östradiol, Melatonin, Serotonin, Katecholamine und /oder Histamin gemessen werden.

### Bewertung:

Cortisol ist hoch, DHEA(S) normal bis niedrig, Testosteron niedrig, Östradiol normal bis erhöht, Serotonin niedrig, Melatonin niedrig, Noradrenalin normal bis hoch, Adrenalin niedrig, Dopamin normal bis niedrig, Histamin erhöht.

### Testbestecks:

Die Testbestecks zur Diagnostik der Adipositas bestehen aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, Testosteron und /oder Östradiol, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA, Glutamat und /oder Histamin.

### Beispiel 12: Prämenstruelles Syndroms (PMS)

Das Verfahren zur Diagnostik des Prämenstruellen Syndroms (PMS) besteht darin, dass Cortisol im Tagesprofil und DHEA /DHEA(S), Testosteron, Östradiol, Progesteron, Melatonin, Serotonin und /oder Katecholamine gemessen werden.

### Bewertung:

Cortisol ist normal, DHEA(S) niedrig, Testosteron normal bis erniedrigt, Östradiol normal bis hoch, Progesteron niedrig, Melatonin niedrig, Serotonin niedrig, Noradrenalin hoch bis normal, Adrenalin niedrig bis normal, Dopamin normal bis erniedrigt.

### Testbestecks:

Die Testbestecks zur Diagnostik des Prämenstruellen Syndroms (PMS) bestehen aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, Testosteron, Östradiol Progesteron, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin und /oder Katecholaminen.

### Beispiel 13: Diagnostik von neuroendokrinen Erkrankungen

Die erfindungsgemäße Diagnostik von neuroendokrinen Erkrankungen geschieht in folgender Weise:
Messung /Testung von Cortisol und DHEA /DHEAS im Speichel, Cortisoltagesprofil im Speichel (morgens 8 Uhr bzw. innerhalb 30 min nach dem Aufstehen; 12 Uhr, gegebenenfalls 16 Uhr und 20 Uhr), Melatonin im Speichel und /oder im Morgenurin, Serotonin im Serum und /oder Speichel, und Neurotransmitter wie Serotonin, Adrenalin, Noradrenalin, Dopamin, Glutamat, GABA, PEA, Glycin, Taurin, Glutamin, Histamin, Aspartat und gegebenenfalls Neurotransmittermetaboliten, insbesondere DOPAC, MHPG, Metanephrine, VMS (Vanillinmandelsäure) oder 5-HIES (5-Hydroxyindolessigsäure, ein Serotoninmetabolit) im Urin (2. Morgenurin).

### Beispiel 14: Weitere Verfahren und Testbestecks

Weitere Verfahren und Testbestecks betreffen die Diagnostik / Bestimmung neuroendokriner gesundheitlicher Störungen, von Neurostresszuständen, von Neuroregulationsstörungen oder von Regulationsstörungen der neuroendokrinen Stressachse und die Erkennung von Erkrankungen der Art Kopfschmerzen, Gelenk- oder Muskelschmerzen, Konzentrationsstörungen, Gedächtnisschwäche (Kurzzeitgedächtnis), Koordinationsstörungen, Klaustrophobie, Reizkolon oder Nahrungsmittelunverträglichkeiten.

### Abkürzungsverzeichnis

- AD: Alzheimer's Desease / Alzheimer-Krankheit
- ADS/ADHS: Aufmerksamkeitsdefizitstörungen, ADS, ADHS
- ADHD: Aufmerksamkeitsdefizitstörungen, ADS, ADHS
- CA: Kanadische Patentschrift
- CFIDS: Chronisches Müdigkeitssyndrom (CFS bzw. CFIDS) Chronic Fatigue Immunodysfunction Syndrome
- CFS: Chronisches Müdigkeitssyndrom (Chronic Fatigue Syndrom)
- CMI: Chronic Multisystem Illnesses
- CRF: Corticotropin-Releasing Faktor
- CRH: Corticotropin-Releasing Hormon
- CRH/NE: CRH/ Norepinephrin (= Noradrenalin - NA)
- CRH/NA: CRH/ Noradrenalin (= Norepinephrin - NE)
- DE-OS: Deutsche Offenlegungsschrift
- DHEA: Dehydroepiandrosteron
- DHEAS: DHEA(S) Dehydroepiandrosteron(sulfat)
- DOPAC: 3,4-Dihydroxyphenylacetic acid
- EDTA: Ethylendiamintetraessigsäure
- EEG: Elektroenzephalographie *(neur)*
- ELISA: Enzyme Linked Immunosorbent Assay, Enzym-Immunoassay
- EP: Europäische Patentschrift
- FMS: Fibromyalgie Syndrom
- GABA: Gamma-Aminobuttersäure
- HCl: Salzsäure
- 5-HIES: 5-Hydroxyindolessigsäure
- HPLC: Hochdruckflüssigkeitschromatographie High Performance / High Pressure Liquid Chromatography
- mcg/g MCS: multiple Chemikalienüberempfindlichkeit (multiple chemical sensitivity)
- MHPG: 3-Methoxy-4-Hydroxy-Phenylglykol
- µmol/g: Mikromol pro Gramm
- ng/ml: Nanogramm pro Milliliter
- OS: Offenlegungsschrift
- PEA: Phenylethylamin
- pg/ml: Pikogramm pro Milliliter
- PMS: Prämenstruelles Syndrom
- RIA: Radioimmunoassay
- SBS: Sick Building Syndrom
- VMA: Vanillinmandelsäure
- VMS: VMA Vanillinmandelsäure
- WIPO: Weltorganisation für Geistiges Eigentum
- WIPO: World Intellectual Property Organization
- WO: Internationale Patentschrift (der WIPO)

## Patentansprüche

1. Verfahren zur Bestimmung von neuroendokrinen gesundheitlichen Störungen, **dadurch gekennzeichnet, dass** Glucocorticoide, Sexualsteroide und Aminosäuren /Neurotransmitter und /oder ihre Metaboliten in Körperflüssigkeiten gemessen werden.

2. Verfahren nach Anspruch 1, **dadurch gekennzeichnet, dass** als
2.1. Glucocorticoide Cortisol oder Corticosteron im Speichel, als Sexualsteroide DHEA /DHEAS [(Dehydroepiandrosteron oder Dehydroepiandrosteron(sulfat) DHEA(S) / DHEAS)], Testosteron, Östradiol und zusätzlich Progesteron oder 17-Hydroxyprogesteron und als Neurotransmitter Katecholamine und ihre Metaboliten, Aminosäuren und ihre Metaboliten im Serum, Speichel oder Urin;
2.2. Katecholamine Adrenalin, Noradrenalin oder Dopamin und als ihre Metaboliten Metanephrin, Normetanephrin, Vanillinmandelsäure (VMA) oder 3-Methoxy-4-Hydroxy-Phenylglykol (MHPG) und als Aminosäuren und ihre Metaboliten Serotonin, Melatonin, Glutamat, Glutamin, Gamma-Aminobuttersäure (GABA), Glycin, Taurin oder PEA (Phenylethylamin)
gemessen werden.

3. Verfahren nach Anspruch 1 und 2, **dadurch gekennzeichnet, dass** die Testung der Glucocorticoide, der Sexualsteroide und von Substanz P im Speichel, von Melatonin im Speichel und /oder im Morgenurin, Serotonin im Urin, Serum und /oder im Speichel, und der Neurotransmitter Serotonin, Adrenalin, Noradrenalin, Dopamin, Glutamat, GABA, Phenylethylamin (PEA), Glycin, Taurin, Glutamin, Histamin, Methylhistamin oder Aspartat im Urin oder im zweiten Morgenurin und /oder zusätzlich die Testung der Neurotransmittermetabolite DOPAC (3,4-Dihydroxyphenylacetic acid), MHPG, Metanephrine, VMS (VMA) oder 5-HIES (5-Hydroxyindol-essigsäure) im Urin oder im zweiten Morgenurin erfolgt.

4. Verfahren nach Anspruch 1 bis 3, **dadurch gekennzeichnet, dass** Speichelmessungen von Cortisol, von DHEA /DHEAS (Dehydroepiandrosterone), Serotonin, Melatonin und /oder von Substanz P und /oder zusätzlich von Testosteron, 17-OH-Progesteron und /oder von Katecholaminen vorgenommen werden.

5. Verfahren nach Anspruch 1 bis 4, **dadurch gekennzeichnet, dass** die Messung der Neurotransmitter
5.1. mittels Enzymimmunoassay vorgenommen wird, so die der Katecholamine, PEA, Serotonin, Histamin, Methylhistamin, Melatonin, GABA oder Glutamat;
5.2. mittels HPLC /Flüssigkeitschromatographie nach Derivatisierung, so für Glycin, Glutamin, Glutaminsäure und /oder Taurin, erfolgt.

6. Verfahren nach Anspruch 1 zur Diagnostik
6.1. von CFS (Chronisches Müdigkeitssyndrom, Chronic Fatigue Syndrom), **dadurch gekennzeichnet, dass** Cortisol im Tagesprofil und DHEA /DHEA(S), Melatonin, Serotonin, Katecholamine, GABA und /oder Glutamat;
6.2. der multiplen Chemikalienüberempfindlichkeit (multiple chemical sensitivity, MCS), **dadurch gekennzeichnet, dass**
6.2.1. Cortisol oder Corticosteron und ferner DHEA /DHEA(S), Melatonin, Serotonin, Substanz P, Katecholamine, GABA, Glutamat, Histamin und /oder Glycin in Körperflüssigkeiten,
6.2.2. Cortisol im Tagesprofil und DHEA /DHEA(S), Melatonin, Serotonin, Katecholamine, GABA und /oder Glutamat,
6.2.3. das Cortisoltagesprofil und DHEA /DHEA(S) im Speichel, Melatonin im Speichel und /oder im Morgenurin, Serotonin im Urin, Serum und /oder im Speichel;
6.3. der Fibromyalgie, **dadurch gekennzeichnet, dass** Cortisol im Tagesprofil und DHEA /DHEA(S), Melatonin, Serotonin, Katecholamine, GABA und /oder Glutamat;
6.4. der Fatigue (zentrale Erschöpfung), **dadurch gekennzeichnet, dass** Cortisol im Tagesprofil und DHEA /DHEA(S), Melatonin, Serotonin, Katecholamine, GABA, Glutamat und /oder Histamin;
6.5. von Aufmerksamkeitsdefizitstörungen (ADS, ADHS), **dadurch gekennzeichnet, dass** Serotonin, Katecholamine, GABA, Glutamat, PEA, Histamin und /oder Kryptopyrrol;
6.6. von Depressionen, **dadurch gekennzeichnet, dass** Cortisol im Tagesprofil und DHEA /DHEA(S), Melatonin, Serotonin, Katecholamine, GABA, Glutamat, PEA, Testosteron und /oder Östradiol und /oder zusätzlich Progesteron;
6.7. des Burn-Out-Syndroms, **dadurch gekennzeichnet, dass** Cortisol im Tagesprofil und DHEA /DHEA(S), Melatonin, Serotonin, Katecholamine, GABA, Glutamat und /oder Testosteron;
6.8. von Schlafstörungen, **dadurch gekennzeichnet, dass** Cortisol im Tagesprofil und DHEA /DHEA(S), Melatonin, Serotonin, Katecholamine, Histamin, Testosteron und /oder Östradiol;
6.9. von Migräne, **dadurch gekennzeichnet, dass** Cortisol im Tagesprofil und DHEA /DHEA(S), Melatonin, Serotonin, Katecholamine, PEA, GABA, Glutamat, Histamin und /oder Glycin;
6.10. von Essstörungen, **dadurch gekennzeichnet, dass** Cortisol im Tagesprofil und DHEA /DHEA(S), Serotonin, Katecholamine, PEA, Histamin, Glycin und /oder GABA;
6.11. der Adipositas, **dadurch gekennzeichnet, dass** Cortisol im Tagesprofil und DHEA /DHEA(S), Testosteron, Östradiol, Melatonin, Serotonin, Katecholamine und /oder Histamin;
6.12. des Prämenstruellen Syndroms (PMS), **dadurch gekennzeichnet, dass** Cortisol im Tagesprofil und DHEA /DHEA(S), Testosteron, Östradiol, Progesteron, Melatonin, Serotonin und /oder Katecholamine
gemessen werden.

7. Verfahren nach Anspruch 1, **dadurch gekennzeichnet, dass** es sich bei den gesundheitlichen Störungen, Neurostresszuständen, Neuroregulationsstörungen oder Regulationsstörungen der neuroendokrinen Stressachse um die Erkrankungen
a) Chronisches Müdigkeitssyndrom (CFS bzw. CFIDS)
b) Multiple Chemikalienüberempfindlichkeit (multiple chemical sensitivity MCS)
c) CMI (Chronic Multisystem Illnesses)
d) Depressionen
e) Burn-Out-Syndrom
f) Fibromyalgie
g) Migräne
h) Schlafstörungen
i) Prämenstruelles Syndrom (PMS)
j) Essstörungen / Appetitstörungen, Heißhunger, Kohlehydrat-Heißhunger
k) Übergewicht / Adipositas
l) Craving
m) Sick-Building Syndrome (SBS)
n) Building related illness
o) Environmental somatisation syndrome
p) Idiopatic Environmental Intolerance
q) Kopfschmerzen
r) Gelenk-, Muskelschmerzen
s) Konzentrationsstörungen, Gedächtnisschwäche (Kurzzeitgedächtnis)
t) Aufmerksamkeitsdefizitstörungen (ADS, ADHS)
u) Koordinationsstörungen
v) Reizkolon
w) Nahrungsmittelunverträglichkeiten
x) Klaustrophobie
y) Regulationsstörungen der neuroendokrinen Stressachse, wie z.B. das Burn-Out-Syndrom oder das Sickness-Syndrom
handelt.

8. Testbestecks zur Bestimmung von neuroendokrinen gesundheitlichen Störungen, bestehend aus Immunoassays und /oder Anordnungen für die Flüssigkeitschromatographie /HPLC zur Messung von Glucocorticoiden, Sexualsteroiden und von Aminosäuren /Neurotransmittern und /oder ihren Metaboliten in Körperflüssigkeiten.

9. Testbestecks nach Anspruch 8, bestehend aus Immunoassays und /oder Anordnungen für die Flüssigkeitschromatographie /HPLC zur Messung /Bestimmung
9.1. der Glucocorticoide Cortisol oder Corticosteron im Speichel, der Sexualsteroide DHEA, DHEAS /DHEA(S), Testosteron, Östradiol, Progesteron oder 17-Hydroxyprogesteron und der Neurotransmitter Katecholamine und ihre Metaboliten, Aminosäuren und ihre Metaboliten im Serum, Speichel oder Urin;
9.2. der Katecholamine Adrenalin, Noradrenalin oder Dopamin und ihrer Metaboliten Metanephrin, Normetanephrin, Vanillinmandelsäure oder 3-Methoxy-4-Hydroxy-Phenylglykol und zur Messung /Bestimmung der Aminosäuren und ihrer Metabolite Serotonin, Melatonin, Glutamat, Glutamin, GABA, Glycin, Taurin oder Phenylethylamin;
9.3. der Glucocorticoide, der Sexualsteroide und von Substanz P im Speichel, von Melatonin im Speichel und /oder im Morgenurin, Serotonin im Urin, Serum und /oder im Speichel, und der Neurotransmitter Serotonin, Adrenalin, Noradrenalin, Dopamin, Glutamat, GABA, PEA, Glycin, Taurin, Glutamin, Histamin, Methylhistamin, Aspartat und der Neurotransmittermetabolite DOPAC, MHPG, Metanephrine, VMS oder 5-HIES im Urin oder im zweiten Morgenurin und die Immunoassays nach Anspruch 9 bis 9.3. Chemolumineszenz-, Enzym- oder Radioimmunoassays darstellen;
9.4. der Steroide im Speichel und von Aminosäuren /Neurotransmittern und ihren Metaboliten im Urin, Serum oder Speichel;
9.5. von Cortisol oder Corticosteron mittels Chemolumineszenz- oder Enzymimmunoassays;
9.6. der Neurotransmitter, bestehend aus
9.6.1. Enzymimmunoassays zur Bestimmung der Katecholamine, PEA, Serotonin, Histamin, Methylhistamin, Melatonin, GABA oder Glutamat,
9.6.2. Anordnungen für die Flüssigkeitschromatographie /HPLC zur Bestimmung von Glycin, Glutamin, Glutaminsäure und /oder Taurin nach Derivatisierung;
9.7. von DHEA /DHEAS, Testosteron, Östradiol oder 17-Hydroxyprogesteron mittels Chemolumineszenz-, Enzym- oder Radioimmunoassays;
9.8. der Katecholamine Dopamin, Adrenalin und /oder Noradrenalin, von GABA, Glutamat, PEA, Histamin, Methyhistamin, Glycin und /oder Taurin und der Metaboliten MHPG, DOPAC, VMS, 5-HIES und /oder der Metanephrine mittels Enzym- oder Radioimmunassays;
9.9. von Melatonin im Speichel und /oder im Morgenurin und von Serotonin im Serum, Urin und /oder Speichel;
9.10. von Cortisol, von DHEA /DHEAS, Serotonin, Melatonin, Substanz P, Testosteron, 17-OH-Progesteron und /oder von Katecholaminen durch Speichelmessungen.

10. Testbestecks nach Anspruch 8 zur Diagnostik
10.1. des Chronischen Müdigkeitssyndroms (CFS, Chronic Fatigue Syndrom), bestehend aus Immunoassays zur Messung /Bestimmung der Steroide im Speichel und aus Immunoassays oder aus Anordnungen für die Flüssigkeitschromatographie /HPLC zur Messung /Bestimmung von Aminosäuren /Neurotransmittern und ihren Metaboliten im Urin, Serum oder Speichel;
10.2. von CFS, bestehend aus Chemolumineszenz- oder Enzymimmunoassays zur Bestimmung von Cortisol im Tagesprofil, das deutlich erniedrigte Werte ausweist und zur Bestimmung von Testosteron, dessen Werte niedrig sein können, aus Chemolumineszenz-, Enzym- oder Radioimmunoassays zur Bestimmung von DHEA /DHEAS mit normalen bis niedrigen Werten für 17-OH-Progesteron, aus Enzymimmunoassays zur Bestimmung von Melatonin, das oft niedrige Werte ausweist, aus Enzym- oder Radioimmunassays oder aus Anordnungen für die Flüssigkeitschromatographie /HPLC zur Bestimmung von Serotonin - mit oft stark erniedrigten Werten, Katecholaminen - mit erhöhten bis stark erniedrigten Werten für Noradrenalin und normalen bis erniedrigten Werten für Dopamin, Adrenalin, GABA und Glutamat;
10.3. von MCS, der multiplen Chemikalienüberempfindlichkeit (multiple chemical sensitivity), bestehend aus
10.3.1. Immunoassays zur Messung /Bestimmung der Steroide im Speichel und aus Immunoassays oder aus Anordnungen für die Flüssigkeitschromatographie /HPLC zur Messung /Bestimmung von Aminosäuren /Neurotransmittern und ihren Metaboliten im Urin, Serum oder Speichel, darunter von Glycin nach Derivatisierung im Urin oder im zweiten Morgenurin mittels Enzym- /Radioimmunassay oder HPLC /Flüssigkeitschromatographie,
10.3.2. Chemolumineszenz- oder Enzymimmunoassays zur Messung /Bestimmung von Cortisol oder Corticosteron im Tagesprofil, aus Chemolumineszenz-, Enzym- oder Radioimmunoassays zur Messung /Bestimmung von DHEA /DHEAS, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Substanz P, Katecholaminen, GABA, Glutamat, Histamin und /oder Glycin in Körperflüssigkeiten, darunter das Cortisol-/ Corticosterontagesprofil und DHEA /DHEA(S) im Speichel, Melatonin im Speichel und /oder im Morgenurin, Serotonin im Urin, Serum und /oder im Speichel;
10.4. der Fibromyalgie, bestehend aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA und /oder Glutamat;
10.5. von Fatigue (zentrale Erschöpfung), bestehend aus Chemolumineszenz- oder Enzymimmunoassays zur Messung /Bestimmung von Cortisol im Tagesprofil, aus Chemolumineszenz-, Enzym- oder Radioimmunoassays zur Bestimmung von DHEA /DHEAS, aus Enzymimmunoassays zur Bestimmung /Messung von Melatonin, aus Enzym- oder Radioimmunassays oder aus Anordnungen für die Flüssigkeitschromatographie /HPLC zur Messung /Bestimmung von Serotonin, Katecholaminen, GABA, Glutamat und /oder Histamin;
10.6. von Aufmerksamkeitsdefizitstörungen (ADS, ADHS), bestehend aus Chemolumineszenz- oder Enzymimmunoassays zur Bestimmung von Cortisol im Tagesprofil, aus Enzym- oder Radioimmunassays oder aus Anordnungen für die Flüssigkeitschromatographie /HPLC zur Messung /Bestimmung von Serotonin, Katecholaminen, GABA, Glutamat, PEA, Histamin und einer photometrischen Anordnung zur Bestimmung /Messung von Kryptopyrrol;
10.7. von Depressionen, bestehend aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS und Testosteron, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA, Glutamat und /oder PEA;
10.8. des Burn-Out-Syndroms, bestehend aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS und Testosteron, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin, Katecholaminen, GABA und /oder Glutamat;
10.9. von Schlafstörungen, bestehend aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS, Testosteron und /oder Östradiol, aus Enzymimmunoassays zur Bestimmung von Melatonin, aus Enzym- oder Radioimmunassays oder aus Anordnungen für die Flüssigkeitschromatographie /HPLC zur Bestimmung von Serotonin und Katecholaminen;
10.10. von Migräne, bestehend aus Chemolumineszenz- oder Enzymimmunoassays zur Bestimmung von Cortisol im Tagesprofil, aus Chemolumineszenz-, Enzym- oder Radioimmunoassays zur Bestimmung von DHEA /DHEAS, aus Enzymimmunoassays zur Bestimmung von Melatonin, aus Enzym- oder Radioimmunassays oder aus Anordnungen für die Flüssigkeitschromatographie /HPLC zur Messung /Bestimmung von Serotonin, Katecholaminen, PEA, GABA, Glutamat, Histamin und /oder Glycin;
10.11. von Essstörungen, bestehend aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, aus Enzym- oder Radioimmunassays oder aus Anordnungen für die Flüssigkeitschromatographie /HPLC zur Messung /Bestimmung von Serotonin, Katecholaminen, PEA, Histamin und /oder Glycin;
10.12. der Adipositas, bestehend aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, Testosteron und /oder Östradiol, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin, Katecholaminen und /oder Histamin;
10.13. des Prämenstruellen Syndroms (PMS), bestehend aus Chemolumineszenz- oder Enzymimmunoassays zur Messung /Bestimmung von Cortisol im Tagesprofil, aus Chemolumineszenz-, Enzym- oder Radioimmunoassays zur Messung /Bestimmung von DHEA /DHEAS, Testosteron, Östradiol Progesteron, aus Enzymimmunoassays zur Messung /Bestimmung von Melatonin, aus Enzym- oder Radioimmunassays oder aus Anordnungen für die Flüssigkeitschromatographie /HPLC zur Messung /Bestimmung von Serotonin und /oder Katecholaminen.

11. Testbestecks nach Anspruch 8, bestehend aus Speichel-, Urin- und /oder Serum-Röhrchen, die Stabilisatoren - EDTA, HCl, Na-Metabisulfit - enthalten, aus Sammelschalen, Pipetten, Sammelanleitungen, Etiketten, standardisiertem Anamnese- und Untersuchungsbogen und aus in den ELISA- oder RIA-Verfahren, der Chemolumineszenz oder der HPLC benötigten spezifischen, markierten Antikörpern.

12. Testbestecks nach Anspruch 8 zur Bestimmung neuroendokriner gesundheitlicher Störungen, bestehend aus Immunoassays zur Messung der Steroide-Werte im Speichel und aus Immunoassays oder aus Anordnungen für die Flüssigkeitschromatographie /HPLC zur Bestimmung von Aminosäuren /Neurotransmittern und ihren Metaboliten im Urin, Serum oder Speichel zur Erkennung von Krankheiten der Art
a) Chronisches Müdigkeitssyndrom (CFS bzw. CFIDS)
b) Multiple Chemikalienüberempfindlichkeit (multiple chemical sensitivity MCS)
c) CMI (Chronic Multisystem Illnesses)
d) Depressionen
e) Burn-Out-Syndrom
f) Fibromyalgie
g) Migräne
h) Schlafstörungen
i) Prämenstruelles Syndrom (PMS)
j) Essstörungen / Appetitstörungen, Heißhunger, Kohlehydrat-Heißhunger
k) Übergewicht / Adipositas
l) Craving
m) Sick-Building Syndrome (SBS)
n) Building related illness
o) Environmental somatisation syndrome
p) Idiopatic Environmental Intolerance
q) Kopfschmerzen
r) Gelenk-, Muskelschmerzen
s) Konzentrationsstörungen, Gedächtnisschwäche (Kurzzeitgedächtnis)
t) Aufmerksamkeitsdefizitstörungen (ADS, ADHS)
u) Koordinationsstörungen
v) Reizkolon
w) Nahrungsmittelunverträglichkeiten
x) Klaustrophobie
y) Regulationsstörungen der neuroendokrinen Stressachse, wie z.B. das Burn-Out-Syndrom oder das Sickness-Syndrom.
